# FASCICULE DE BREVET EUROPEEN

(11) **EP 3 923 814 B1**
(45) Date de publication et mention de la délivrance du brevet: **19.11.2025**
(21) Numéro de dépôt: 20704034.6
(22) Date de dépôt: 14.02.2020
(51) Int. Cl.: A61B 8/00, A61B 8/08, A61B 5/00, A41C 3/00, A61B 5/01

(54) **PIÈCE D'HABILLEMENT POUR CONTRÔLE DE CELLULES**
BEKLEIDUNGSSTÜCK ZUR KONTROLLE VON ZELLEN
ARTICLE OF CLOTHING FOR CONTROLLING CELLS

(30) Priorité: 15.02.2019 EP 19157593
(43) Date de publication de la demande: 22.12.2021
(73) Titulaire: Boysset, Max, 1427 Bonvillars (CH)
(72) Inventeur: Boysset, Max, 1427 Bonvillars (CH)
(74) Mandataire: Omnis-IP
(86) Numéro de dépôt international: PCT/EP2020/053831
(87) Numéro de publication internationale: WO 2020/165383

(56) Documents cités:
- WO-A1-2018/060456
- WO-A2-2016/196741
- US-A1- 2011 087 096
- US-A1- 2013 023 767
- US-A1- 2013 317 364
- US-A1- 2016 303 402
- US-A1- 2018 296 859

## Description

### DOMAINE TECHNIQUE

La présente invention concerne une pièce d'habillement destinée à être portée par un utilisateur de façon à couvrir une partie du corps de cet utilisateur.

Une telle pièce d'habillement peut en particulier être utilisée dans le cadre de la prévention, de la détection et/ou du traitement de certaines pathologies et en particulier de cancers. Cette pièce d'habillement peut notamment être un sous-vêtement.

### ART ANTÉRIEUR

Actuellement, il existe peu de moyens efficaces et éprouvés pour la prévention de certaines pathologies telles que les cancers et notamment le cancer du sein, le cancer des testicules ou le cancer colorectal. Généralement, la prévention se limite à minimiser les facteurs de risque tels que la fumée, l'alcool, le rayonnement UV ou une nourriture malsaine notamment. La maladie est prise en charge au moment où elle est déclarée et détectée, ce qui nécessite souvent une prise en charge drastique, qui peut être douloureuse et particulièrement pénible pour le patient.

Il serait souhaitable de disposer d'une solution de prévention des cancers qui soit peu contraignante, facile à mettre en œuvre et aussi peu dérangeante que possible pour l'utilisateur.

En ce qui concerne la détection de cancers, en particulier de cancers du sein, une mammographie est généralement préconisée à intervalles réguliers, par exemple chaque année ou tous les deux ans, aux femmes ayant atteint un certain âge. Les mammographies étant douloureuses, contraignantes et désagréables, toutes les femmes ne sont pas prêtes à les faire régulièrement. Par ailleurs, un tel examen nécessite d'organiser un rendez-vous et de se rendre sur le lieu du contrôle, ce qui présente une contrainte qui n'incite pas toutes les femmes à pratiquer cet examen régulièrement.

Dans le cas où un cancer se développe peu de temps après un examen, il peut se passer une longue période avant qu'un nouvel examen ne soit effectué et que le cancer soit détecté. Or il est bien connu que plus un cancer est détecté et traité à un stade précoce, plus les chances de guérison sont grandes.

Il existe donc un besoin pour un moyen de détection non contraignant, indolore et utilisable régulièrement pour la détection de cancers du sein en particulier, mais d'autres cancers également.

En ce qui concerne le traitement de cancers, notamment de cancers du sein, les moyens de traitement sont généralement lourds et très invasifs. Ces moyens de traitement sont notamment la radiothérapie, la chimiothérapie et/ou l'ablation du sein. Il existe également des thérapies ciblées, mais qui ne sont possibles que pour certains types de cancers spécifiques.

Ces thérapies étant pratiquées en milieu hospitalier, les patientes doivent se déplacer et sont traitées pendant une période relativement courte, avec des doses de médicaments relativement fortes. Ces fortes doses de médicaments entraînent des effets secondaires qui peuvent être importants et particulièrement désagréables.

Il existe donc un besoin pour une thérapie aussi peu invasive que possible, utilisant des doses de produits thérapeutiques moins importantes, mais réparties sur une période plus longue de façon à minimiser les effets secondaires.

Les publications WO2018/060456A1, US 2013/317364 et US 2013/0023767 décrivent toutes les deux, une pièce d'habillement réalisée sous la forme d'un soutien-gorge et destinée à la détection précoce d'une tumeur du sein.

A cet effet, le soutien-gorge comporte un générateur de signaux, par exemple d'ultrasons, et des récepteurs de signaux. Il comporte en outre un dispositif de traitement des signaux reçus par les récepteurs.

Lorsque le soutien-gorge est porté par une utilisatrice, le générateur de signaux émet des signaux en direction des seins de cette utilisatrice. Ces signaux sont diffusés et réfléchis par les tissus biologiques des seins, puis certains d'entre eux sont captés par les récepteurs du soutien-gorge. Les signaux ainsi captés sont traités par le dispositif de traitement de façon à déterminer si les seins de l'utilisatrice contiennent des tissus biologiques anormaux tels que des tumeurs ou des kystes.

En cas de détection d'un tel tissu biologique anormal, une alarme est déclenchée, de façon à informer l'utilisatrice de la présence d'un tel élément anormal et de lui indiquer qu'une consultation médicale est recommandée.

Il est clair que les soutiens-gorge décrits dans ces documents ne sont utiles et utilisables que lorsqu'un tissu anormal tel qu'une tumeur ou un kyste est formé. Un tel soutien-gorge est inutile dans le cadre de la prévention d'un cancer puisqu'en l'absence de tissu anormal, il ne donne aucune information.

De même, ce soutien-gorge est également inutile dans le cadre d'un traitement d'un cancer. En effet, la fonction du soutien-gorge étant uniquement d'informer en cas de détection d'un tissu anormal, il n'aurait aucune utilité si la présence d'un tel tissu anormal est déjà connue.

Le document US 2016 303402 décrit un dispositif portable destiné à moduler l'activité de cellules humaines par l'application d'ultrasons. Ce dispositif est essentiellement utilisé pour modifier l'activité de cellules d'un patient, par exemple pour favoriser la sécrétion d'insuline. Ce dispositif peut également être utilisé pour traiter une tumeur du cerveau. Ce dispositif ne concerne toutefois pas la prévention de tumeurs et ne peut pas être utilisé de façon autonome, en ce sens que le même dispositif ne peut pas être utilisé pour détecter une tumeur et la traiter. De manière similaire, le dispositif ne peut pas être utilisé pour déterminer qu'aucune cellule anormale n'est présente et passer en mode de prévention.

Il existe donc un besoin pour une pièce d'habillement qui soit utilisable non seulement pour détecter l'existence de cellules malsaines, mais également pour prévenir l'apparition de telles cellules malsaines et/ou pour traiter ces cellules malsaines après leur apparition.

### DESCRIPTION DE L'INVENTION

Une telle pièce d'habillement est décrite dans la présente invention.

Le but de l'invention est atteint par une pièce d'habillement destinée à être portée par un utilisateur de façon à couvrir une partie du corps de cet utilisateur, cette pièce d'habillement comportant un élément d'habillement souple recouvrant ladite partie du corps de l'utilisateur, ladite pièce d'habillement comportant en outre un générateur de signaux électriques et au moins un transducteur solidaire dudit élément d'habillement souple, ledit au moins un transducteur étant agencé pour convertir lesdits signaux électriques du générateur de signaux électriques en ultrasons et pour transmettre les ultrasons générés par ce transducteur à ladite partie du corps de l'utilisateur couverte par cet élément d'habillement souple, cette pièce d'habillement étant caractérisée en ce que ledit au moins un transducteur est agencé pour émettre des ultrasons ayant au moins trois intensités acoustiques différentes comprises dans au moins trois plages d'intensités distinctes, l'une desdites plages d'intensité correspondant à un mode de prévention destiné à prévenir l'apparition de cellules biologiques anormales, une autre plage d'intensité correspondant à un mode de détection destiné à détecter de telles cellules anormales, et une troisième plage d'intensités correspondant à un mode de traitement destiné à l'élimination de telles cellules anormales et au traitement de la pathologie, en ce que cette pièce d'habillement comporte au moins un récepteur agencé pour recevoir des ondes provenant dudit au moins un transducteur après réflexion par ladite partie du corps de l'utilisateur recouverte par ladite pièce d'habillement et pour transformer ces ondes en signaux électriques, en ce que cette pièce d'habillement comporte un module de communication agencé pour transmettre ces signaux électriques à une unité de traitement agencée pour en extraire une information, en ce que cette pièce d'habillement comporte un récepteur de commande agencé pour recevoir un message de commande dépendant de ladite information extraite par l'unité de traitement et pour transmettre ce message de commande audit générateur de signaux, ce générateur de signaux étant agencé pour activer ledit au moins un transducteur selon le contenu du message reçu, le mode de prévention étant activé si aucune cellule anormale n'est détectée lorsque ledit au moins un transducteur est utilisé en mode de détection, le mode de traitement étant activé si des cellules anormales sont détectées lorsque ledit au moins un transducteur est utilisé en mode de détection, et en ce que ces modes d'utilisation sont gérés de façon totalement autonome et automatique.

La pièce d'habillement de l'invention permet de mettre en œuvre un mode de prévention de maladies et en particulier de cancers, sans que l'utilisateur ne soit dérangé ou perturbé. Cette prévention peut se faire sans aucune gêne et sans contrainte pour l'utilisateur, en particulier à son domicile ou à n'importe quel moment de sa vie courante. Ceci permet de mettre en œuvre la prévention pendant de longues durées, contrairement à ce qui serait possible par exemple si la prévention devait se faire dans un milieu spécialisé tel qu'un milieu hospitalier. Du fait de cette longue durée, la prévention peut être d'autant plus efficace.

La pièce d'habillement peut être adaptée au type de prévention souhaité et à la partie du corps de l'utilisateur que la personne concernée souhaite prendre en compte. Cette pièce d'habillement peut notamment être un soutien-gorge, un slip, un collant, une chemisette, un débardeur, une cagoule ou un bonnet par exemple.

La pièce d'habillement de l'invention peut également être utilisée pour la détection de cellules anormales. Cette détection peut se faire très régulièrement, par exemple une fois par jour ou une fois par semaine, sans même que le porteur de la pièce d'habillement ne s'en rende compte, c'est-à-dire sans aucune gêne ou désagrément pour l'utilisateur.

Cette détection quotidienne ou hebdomadaire ou selon toute autre fréquence choisie, permet de comparer les résultats obtenus un certain jour aux résultats obtenus lors de contrôles précédents et permet ainsi de suivre l'évolution de la partie du corps de l'utilisateur. Il est ainsi possible de détecter très rapidement une évolution ou une modification anormale et d'agir en conséquence.

La pièce d'habillement peut également être utilisée à titre curatif. Dans ce cas, elle peut être adaptée spécialement à l'utilisateur, à sa pathologie et aux endroits que l'utilisateur souhaite traiter.

Comme la pièce d'habillement peut être portée par l'utilisateur de la même façon qu'un habit conventionnel et qu'il ne présente aucune gêne ou aucun inconfort, il est possible de porter cet habit pendant une longue période de la journée, typiquement plusieurs heures par jour, tous les jours. Ceci permet d'appliquer un traitement selon un dosage nettement moins élevé que lorsque le traitement est appliqué pendant une période très courte, comme par exemple une heure par semaine, ce qui serait typiquement le cas en milieu hospitalier.

Les effets secondaires qui peuvent être induits par des fortes doses de traitement peuvent être réduits voire supprimés par l'application de doses plus faibles, pendant une durée plus longue. Le fait de pouvoir utiliser le dispositif de l'invention pendant des périodes très longues par rapport aux traitements conventionnels ouvre la porte à des traitements différents et en particulier à des traitements qui n'auraient aucun effet ou un effet très faible s'ils étaient appliqués pendant une durée courte. Ces traitements ou ces thérapies peuvent potentiellement ne pas avoir d'effet secondaire ou des effets secondaires moindres que les thérapies conventionnelles.

### BRÈVE DESCRIPTION DES DESSINS

La présente invention et ses avantages seront mieux compris en référence aux figures annexées et à la description détaillée d'un mode de réalisation particulier, dans lesquelles :
- la figure 1 illustre schématiquement un mode de réalisation particulier d'une pièce d'habillement selon l'invention ;
- la figure 2 est une vue schématique en coupe d'une partie de la pièce d'habillement de la figure 1 ;
- la figure 3 représente un premier mode d'utilisation de la pièce d'habillement de l'invention ;
- la figure 4 illustre un second mode d'utilisation de la pièce d'habillement de l'invention ;
- la figure 5 est une variante du mode de réalisation illustré par la figure 4 ; et
- la figure 6 représente un troisième mode d'utilisation de la pièce d'habillement de l'invention.

### MODES DE REALISATION AVANTAGEUX

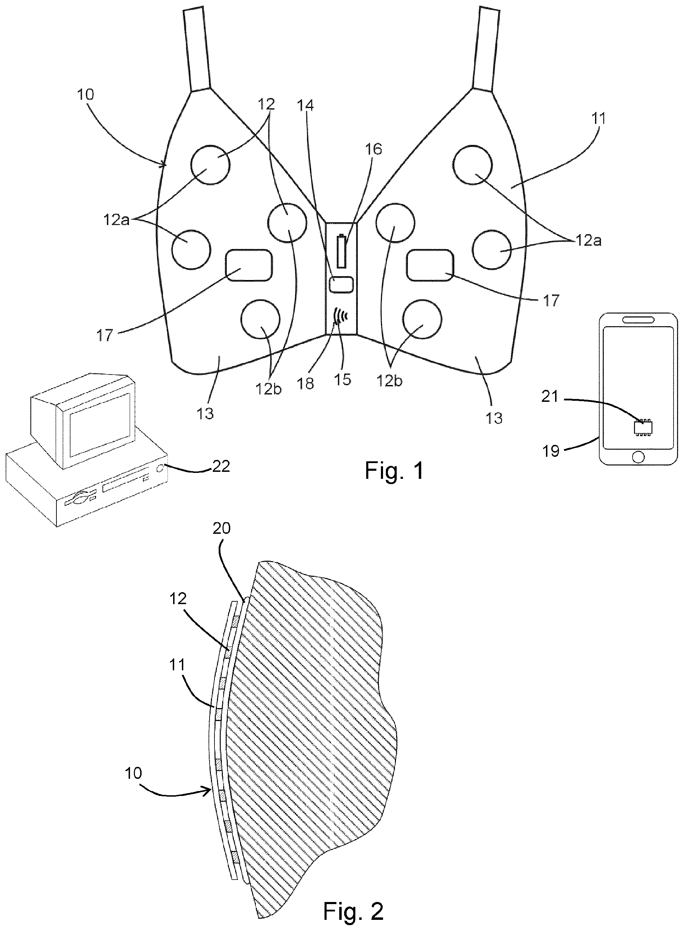

En référence aux figures 1 et 2, la pièce d'habillement 10 est représentée sous la forme d'un soutien-gorge. Il pourrait toutefois avoir d'autres formes en fonction de la destination prévue de cette pièce d'habillement.

La pièce d'habillement comporte un élément d'habillement souple 11 réalisé généralement en tissu et destiné à épouser la forme de la peau de l'utilisateur, à être à proximité de la peau de l'utilisateur et à recouvrir une partie du corps de cet utilisateur.

Cette pièce d'habillement 10 comporte au moins un émetteur 12a d'ondes ultrasonores, fixé à l'élément d'habillement souple 11. La pièce d'habillement comporte également au moins un récepteur 12b d'ondes réfléchies par des tissus biologiques du porteur de la pièce d'habillement, ces ondes provenant de la réflexion des ondes ultrasonores émises par ledit au moins un émetteur 12a.

Dans un mode de réalisation avantageux, chaque émetteur 12a est formé d'un transducteur 12 ayant pour fonction de transformer des signaux électriques en ondes ultrasonores. Chaque récepteur 12b est également formé d'un transducteur 12 ayant pour fonction de transformer des ondes réfléchies par les tissus biologiques de l'utilisateur en signaux électriques. Ces transducteurs peuvent être physiquement les mêmes éléments jouant alternativement le rôle d'émetteurs et de récepteurs, ou être de la même nature, certains d'entre eux étant utilisés comme émetteurs et d'autres comme récepteurs, ou encore être de nature différente, les transducteurs d'un type étant utilisés comme émetteur et les transducteurs d'un autre type étant utilisés comme récepteurs.

Ces transducteurs 12 sont de préférence des éléments ayant une dimension relativement faible et sont réalisés de manière à être le moins gênant possible pour l'utilisateur.

Dans le cas pratique où la pièce d'habillement est un soutien-gorge, celui-ci est formé de deux bonnets 13 qui supportent chacun au moins un transducteur 12. Dans l'exemple illustré par la figure 1, chaque bonnet supporte quatre transducteurs 12 disposés dans différents endroits de ces bonnets. Ces transducteurs peuvent avoir une forme galbée et/ou être souples, de façon à s'adapter au mieux à la forme du soutien-gorge et à ne pas être dérangeant pour la personne qui porte ce soutien-gorge.

La pièce d'habillement de l'invention comporte un générateur 14 de signaux électriques connecté aux transducteurs 12. Ce générateur de signaux 14 peut être maintenu par l'élément d'habillement souple 11 ou au contraire, être disposé à côté de cet élément souple. Dans le mode de réalisation illustré, le générateur 14 de signaux électriques est disposé sur l'élément d'habillement souple 11, entre les deux bonnets 13.

Ce générateur de signaux 14 est en charge d'activer ou non les différents transducteurs 12, selon des modalités décrites plus en détail plus bas.

Les transducteurs 12 sont prévus pour recevoir des signaux électriques du générateur de signaux 14 et pour transformer ces signaux électriques en ultrasons. Dans ce mode de réalisation, les transducteurs jouent le rôle d'émetteurs. Les ultrasons générés peuvent avoir différentes fréquences, différentes amplitudes, différentes puissances ou différentes intensités acoustiques notamment. Il est possible de prévoir que tous les transducteurs émettent des ultrasons ayant les mêmes caractéristiques ou au contraire, qu'ils émettent des ultrasons ayant des caractéristiques physiques différentes.

Le générateur 14 de signaux électriques peut être prévu pour être réglable de façon à modifier au moins certaines caractéristiques des ondes générées par les transducteurs, sous le contrôle du générateur.

Selon un mode de réalisation concret, les ultrasons utilisés peuvent avoir une fréquence comprise entre 600 kHz et 7 MHz et une intensité acoustique variant de 0.01 à plus de 10 W/cm² selon le mode d'utilisation choisi.

Selon un mode de réalisation concret, les transducteurs 12 sont formés d'éléments piézoélectriques ayant une fréquence de résonnance de l'ordre de 2 MHz. Ils sont réalisés en PZT (Titano-Zirconate de Plomb) et ont une taille de 5 x 5 mm et une épaisseur de 1 mm. Chaque bonnet comporte 512 transducteurs piézoélectriques, ce qui permet de détecter un amas de cellules de taille inférieure ou proche de 1 mm de diamètre. Une telle taille correspond à un stade précoce de l'amas de cellules cancéreuses.

Une fois les ultrasons générés par les transducteurs 12 ou les éléments piézoélectriques, ils doivent être transférés dans le corps humain. L'impédance acoustique du corps humain et des transducteurs est respectivement de 1,5 MRayl et 35 MRayl. Cette différence d'impédance rend la transmission des ultrasons entre les deux milieux très peu efficace. Avec les valeurs typiques de l'impédance acoustique des deux milieux, le coefficient de transmission est inférieur à 0,1%, ce qui signifie qu'une grande partie des ondes ultrasonores est perdue à l'interface. Une couche d'adaptation entre les transducteurs et la peau est avantageusement utilisée pour adapter l'impédance.

Une couche d'adaptation ayant une impédance d'environ 10 MRayl est optimale dans un système à trois couches, à savoir les transducteurs, l'élément d'adaptation d'impédance et la peau de l'utilisatrice.

Comme pour une échographie conventionnelle, il est possible d'appliquer un gel d'adaptation d'impédance directement sur la peau de l'utilisatrice. Cette solution n'est toutefois pas optimale dans le cas d'une pièce d'habillement pour des questions de simplicité d'utilisation et pour éviter le nettoyage du gel après usage. Cette solution peut toutefois quand même être utilisée en pratique.

Une solution préférée est l'utilisation d'un élément d'adaptation d'impédance 20 solide, souple, qui puisse être placé dans la pièce d'habillement, comme cela est illustré par la figure 2.

Dans un mode de réalisation concret pour la réalisation d'un élément d'adaptation d'impédance, deux méthodes ont été mises en pratique. La première consiste à créer un hydrogel à base de diacrylate de PEG (poly(éthylène glycol) diacrylate) avec des propriétés ajustables afin d'obtenir une impédance d'environ 10 MRayl. Pour obtenir un hydrogel flexible ayant un diamètre d'environ 20 cm et une épaisseur de 5 mm, les concentrations suivantes ont été utilisées : PEGDA700 (12,5 g. 700 g.mol⁻¹) ; H₂O (37,5 g) et 500 µL d'un photo-initiateur (2-Hydroxy-2-méthylproplophénone, 97%) et un temps de durcissement de 30 minutes sous lumière UV après dégazage à l'azote.

La deuxième méthode testée utilise du silicone. Pour obtenir un gel solide, le silicone doit être placé dans un réseau polymère. Pour ce faire, un gel PDMS (Polydiméthylsiloxane) a été réalisé avec un élastomère de silicone (35 g) et une réticulation d'élastomère de silicone (3,5 g), durci pendant 10 h à 70 °C après dégazage sous vide.

Un matériau de support est fixé sur l'électrode opposée au gel PDMS afin de prévenir les vibrations excessives du transducteur. La réduction des vibrations excessives implique que le transducteur génère des ondes ultrasonores avec une longueur d'impulsion plus courte, améliorant la résolution axiale dans les images. Cependant, un compromis entre résolution axiale et sensibilité, qui est liée à la tension de sortie, doit être fait. La résolution axiale donne la résolution spatiale et le niveau de sensibilité donne le niveau de bruit dans l'image finale.

La pièce d'habillement 10 de l'invention comporte un récepteur de commandes 15, qui peut être intégré au générateur 14 de signaux électriques ou qui peut en être séparé. Ce récepteur de commandes 15 est agencé pour recevoir des commandes provenant par exemple d'une application gérée par un dispositif externe 19, tel qu'un smartphone hébergeant une application dédiée. Lorsqu'un message de commande est reçu par le récepteur de commandes 15, il est traité pour être ensuite transmis au générateur de signaux 14 qui va à son tour activer le ou les transducteurs 12 selon le contenu du message reçu. Un tel message peut par exemple indiquer lequel ou lesquels des transducteurs 12 doivent être activés, parmi tous les transducteurs disponibles, quelle est l'amplitude, l'intensité et/ou la puissance des ondes ultrasonores à générer, éventuellement leur fréquence, pour autant que la fréquence puisse être réglée, la durée, des paramètres de cycle, etc.

Comme mentionné, le récepteur de commandes 15 peut recevoir des commandes provenant du dispositif 19 externe à la pièce d'habillement. Il peut toutefois également recevoir des informations provenant de la pièce d'habillement elle-même. Ces informations peuvent en particulier concerner les transducteurs 12 et indiquer quels transducteurs sont actifs, l'amplitude des ondes ultrasonores générées, leur fréquence, leur intensité, le temps d'exposition, des paramètres de cycle, etc.

La pièce d'habillement 10 peut également comporter une alimentation en énergie 16, généralement réalisée sous la forme d'une batterie qui peut elle aussi être placée sur la pièce d'habillement 10 ou à côté de celle-ci. Selon un mode de réalisation préféré, cette batterie peut être chargée au moyen d'une connexion de type USB et avoir une autonomie si possible d'au moins 24 heures.

La pièce d'habillement peut en outre comporter un ou plusieurs dispositifs de mesure 17 destiné à mesurer certains paramètres liés à la personne qui porte cette pièce d'habillement. A titre d'exemple, le dispositif de mesure 17 pourrait mesurer la température à certains endroits du corps de l'utilisateur en contact avec la pièce d'habillement.

Les informations envoyées au récepteur de commande 15 peuvent également provenir du dispositif de mesure 17 et concerner les paramètres mesurés par ce dispositif de mesure.

Le récepteur de commandes 15 peut comporter un module de communication 18 agencé pour transmettre les informations récoltées à un organe externe tel qu'une mémoire, un dispositif ou un réseau de communication notamment.

Une fois émises par les transducteurs jouant le rôle d'émetteurs, les ondes ultrasonores traversent l'élément d'adaptation d'impédance 20 et se propagent dans différents tissus biologiques. Les différences de densités des différents tissus biologiques correspondent à différentes impédances. Ces différences d'impédance créent un écho qui est capté par les transducteurs. Dans ce contexte, les transducteurs 12 jouent le rôle de récepteurs 12b. Il est à noter que les transducteurs utilisés comme émetteurs 12a peuvent être également ceux utilisés comme récepteurs. Il est également possible d'utiliser des transducteurs différents comme émetteurs et comme récepteurs, ces transducteurs étant toutefois réalisés de la même manière. Il est aussi possible d'utiliser des transducteurs différents comme émetteurs et comme récepteurs, ces transducteurs étant réalisés de manière différente en fonction de leur rôle. Il est également possible d'utiliser différents transducteurs comme émetteurs, en fonction des caractéristiques physiques des ondes émises et en particulier de leur intensité acoustique et également différents transducteurs comme récepteurs, en fonction des caractéristiques physiques des ondes reçues.

Les transducteurs 12 convertissent les ondes de l'écho en signaux électriques. Ces signaux électriques sont traités de façon à extraire les informations relatives aux tissus biologiques. Ce processus permet de créer une image échographique des tissus biologiques sur lesquels ces ondes sont réfléchies. Plus un tissu organique est dense, plus les ondes ultrasonores seront réfléchies. Ainsi, les transducteurs détecteront un son réfléchi lorsque les ultrasons rebondiront sur une masse tissulaire. Plusieurs transducteurs sont utilisés pour créer une image des tissus mammaires.

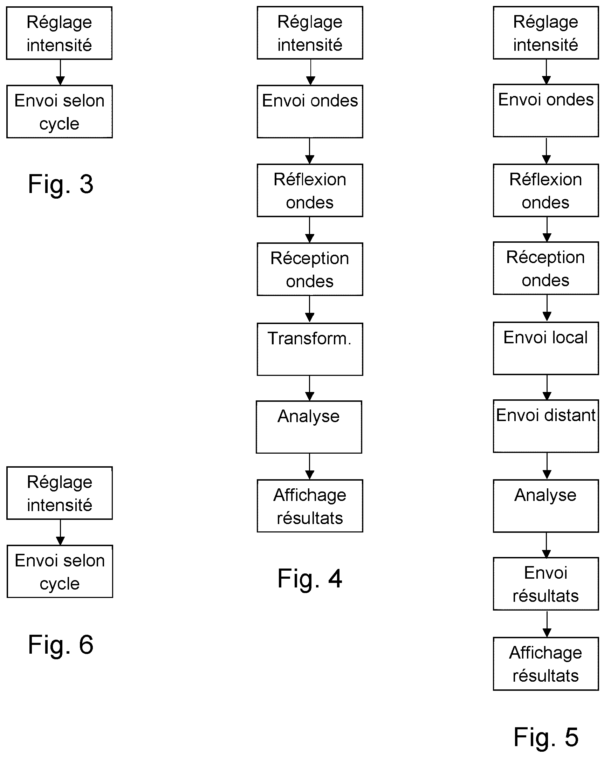

Trois modes d'utilisation de la pièce d'habillement de l'invention sont décrits en référence aux figures 3 à 6.

Comme cela est connu, les cellules biologiques normales peuvent être soumises à un processus connu sous le nom d'apoptose. Ce processus est celui par lequel des cellules déclenchent leur autodestruction en réponse à un signal. Dans le cas de cellules cancéreuses, ce processus d'apoptose n'est pas réalisé et des cellules malsaines, qui devraient normalement être éliminées par le corps, ne sont pas détruites. Il a été montré que l'application d'ondes ultrasonores sur un tissu biologique peut avoir différents effets, selon l'intensité acoustique de ces ondes ultrasonores. Dans certaines plages d'intensité, les ondes ultrasonores ont la faculté de régénérer le processus d'apoptose des cellules.

Le premier mode d'utilisation décrit concerne la prévention et en particulier la prévention du cancer du sein ou d'une autre partie du corps sur lequel la pièce d'habillement est placée.

Dans ce mode d'utilisation illustré par la figure 3, l'intensité acoustique des ondes ultrasonores est tout d'abord sélectionnée. Cette intensité est choisie entre 0.05 et 2 W/cm². La fréquence des ondes ultrasonores utilisées peut être choisie entre environ 600 kHz et 7 MHz. Cette fréquence peut être sensiblement la même dans tous les modes d'utilisation ou peut dépendre du mode d'utilisation, tout en restant de préférence dans la plage telle que définie ci-dessus. Les caractéristiques physiques des ondes ultrasonores sont choisies de façon à être compatibles avec la fonction de régénération de l'apoptose.

En ce qui concerne toujours la prévention, les ondes peuvent être émises selon un cycle prédéfini. Un cycle utilisable en pratique est par exemple l'envoi d'ondes ultrasonores pendant une durée de 2 minutes, suivi d'une pause de 15 minutes, le cycle étant par exemple répété 10 fois pendant une journée.

En principe, dans le cadre de la prévention, les transducteurs 12 des deux bonnets 13 sont utilisés de façon symétrique et tous les transducteurs sont utilisés. D'autres modes de réalisation sont possibles, par exemple l'utilisation des transducteurs d'un seul bonnet à la fois ou l'utilisation seulement d'une partie des transducteurs.

Un deuxième mode d'utilisation illustré par les figures 4 et 5 concerne la détection de de cellules anormales et en particulier de cellules cancéreuses. La figure 4 illustre un premier mode de réalisation de ce mode d'utilisation. Dans celui-ci, l'intensité des ondes ultrasonores est sélectionnée de façon à être comprise entre 1 et 3 W/cm². Les caractéristiques physiques des ondes ultrasonores sont choisies de façon à être compatibles avec une application d'échographie.

Des ondes sonores sont émises par les transducteurs 12, traversent l'élément d'adaptation d'impédance 20 et sont transmises aux tissus biologiques de l'utilisatrice. Certaines des ondes émises sont réfléchies par ces tissus, en particulier par l'interface entre des tissus biologiques ayant des densités différentes.

Les ondes réfléchies par les cellules de l'utilisateur sont captées par transducteurs 12 ayant la fonction de récepteurs et sont transformées en signaux électriques par ces transducteurs. Ces signaux électriques sont ensuite transmis à une unité de traitement 21 par le module de communication 18 placé sur la pièce d'habillement.

Selon un premier mode de réalisation, cette unité de traitement 21 peut elle aussi être placée directement sur la pièce d'habillement 10. Ceci implique toutefois une alimentation adaptée et une dimension de l'unité de traitement compatible avec le port sur la pièce d'habillement.

Selon un deuxième mode de réalisation, l'unité de traitement 21 est séparée de la pièce d'habillement. Cette unité de traitement 21 peut être intégrée au dispositif externe 19, ce dernier pouvant être réalisé sous la forme d'un smartphone ou d'un équipement similaire comportant une application ou un logiciel de traitement de signaux et des moyens de réception des signaux mesurés par les capteurs de la pièce d'habillement.

L'unité de traitement 21 reçoit des données du dispositif externe 19, celui-ci comportant un récepteur adapté pour recevoir les signaux émis par le module de communication 18. Ces signaux sont ensuite traités par l'unité de traitement 21 de façon à en extraire une information et/ou une image.

Lorsque les données ont été traitées par l'unité de traitement 21, un résultat peut être mis à disposition de l'utilisatrice sur la pièce d'habillement au moyen d'une alarme sonore, d'une vibration ou d'une alarme lumineuse indiquant que le détecteur n'a rien relevé d'anormal ou indiquant au contraire qu'un médecin devrait être consulté.

Le résultat peut ainsi être mis à disposition sur le dispositif externe 19, par exemple sur un écran du smartphone, sous forme d'image, de texte, de son ou de mouvement. Il est également possible de n'afficher un résultat que si le traitement des mesures a permis de relever des cellules anormales et de ne rien indiquer si aucune cellule anormale n'a été détectée.

L'information donnée à l'utilisateur/trice peut donc être la présence ou l'absence de cellules anormales. Cette information peut être délivrée sur la base de la comparaison des signaux avec des informations d'une base de données mémorisée par exemple dans l'unité de traitement 21. L'image peut être une image en trois dimensions de l'intérieur des seins de l'utilisatrice, avec par exemple la mise en évidence d'amas de cellules anormales.

Selon un troisième mode de réalisation illustré par la figure 5, l'étapes du choix de l'intensité acoustique, de l'émission d'ondes ultrasonores par les transducteurs, de réflexion des ondes ultrasonores par les tissus biologiques et de réception des ondes par les récepteurs/transducteurs sont les mêmes que ceux décrits en référence à la figure 4. Les signaux électriques des récepteurs/transducteurs sont également transmis à un dispositif externe 19, celui-ci étant de préférence externe à la pièce d'habillement 10.

Dans ce mode de réalisation, le dispositif externe 19 n'héberge pas d'unité de traitement et ne dispose pas nécessairement de moyens de traitement de données. Par contre, ce dispositif externe 19 dispose d'une voie de communication à distance avec un centre de traitement 22 distant. Dans ce cas, les signaux captés par les transducteurs/détecteurs de la pièce d'habillement sont transmis localement au dispositif externe 19. Ces signaux sont ensuite transmis à distance au centre de traitement 22 distant qui prend en charge ce traitement.

A titre d'exemple, le centre de traitement 22 peut former une image 3D des cellules, puis comparer cette image 3D à une base de données d'images correspondant à des cellules saines et/ou des cellules malsaines. Cette comparaison permet de déterminer si les cellules de la personne sur laquelle les mesures ont été faites correspond à des cellules saines ou malsaines. Cette comparaison peut typiquement être améliorée par l'utilisation de techniques d'intelligence artificielle et d'apprentissage profond (deep learning).

Il est également possible de stocker les images de chaque détection correspondant à une personne déterminée, puis de comparer les images les unes aux autres. De cette façon, il est possible de détecter une évolution ou une modification de la structure des tissus biologiques pour la personne en question. Si cette évolution est anormale ou si les cellules correspondent à des cellules malsaines, il est possible d'avertir la personne à qui ces images correspondent et de lui recommander une visite médicale.

Il est également possible de recueillir les données d'un grand nombre de personnes et de les associer au qualificatif de sain ou malsain, de façon à former une base de connaissances et d'améliorer petit à petit la fiabilité du traitement des images.

La phase de détection de cellules anormales peut être faite par exemple quotidiennement, de façon hebdomadaire ou à n'importe quelle fréquence choisie. Cette phase peut nécessiter une durée de quelques dizaines de secondes à quelques minutes. Une durée de deux minutes est généralement suffisante pour effectuer une phase complète de détection.

Selon un troisième mode d'utilisation illustré par la figure 6, la pièce d'habillement est utilisée pour le traitement d'une pathologie telle qu'un cancer. Dans ce mode d'utilisation, l'intensité des ondes ultrasonores est généralement supérieure à 10 W/cm². Les caractéristiques physiques des ondes ultrasonores sont choisies de façon à permettre la destruction de certaines cellules. Les ondes ultrasonores ne sont pas nécessairement émises par tous les transducteurs, mais peuvent au contraire être émises par des transducteurs ciblés, placés à proximité de zones où des cellules anormales ont été détectées. Ces ondes sont émises selon des cycles spécifiques qui peuvent dépendre de la localisation des cellules, de leur extension, d'un choix du médecin ou d'autres paramètres. A titre d'exemple, le traitement pourrait comprendre trois cycles de 1 minutes, suivies de 15 minutes de pauses, à répéter cinq fois pendant la journée.

La pièce d'habillement selon l'invention permet de préférence les trois modes d'utilisation que sont la prévention, la détection et le traitement. La détection est initiée par exemple une fois par jour, avec comme résultat, aucune indication si aucune cellule anormale n'a été détectée et une alarme si des cellules anormales ont été détectées. En cas d'absence de cellules anormales, le mode de prévention peut être utilisé, selon un cycle préfini.

En cas de détection de cellules anormales, généralement, une alarme est déclenchée pour indiquer à l'utilisateur qu'un avis médical est recommandé. Dans le cas où un traitement est requis, le mode de traitement peut être activé selon un cycle prédéfini. Le mode de détection permet de surveiller, également en cas de traitement, l'évolution des cellules anormales et éventuellement d'adapter le traitement en conséquence. L'adaptation du traitement peut concerner l'intensité des ondes utilisées, la durée d'un cycle, le nombre de cycle par unité de temps, les transducteurs activés, etc.

Le mode d'utilisation à mettre en œuvre est choisi en sélectionnant l'intensité acoustique des ondes ultrasonores émises. Cette intensité peut être choisie parmi au moins deux plages d'intensités distinctes, mais de préférence parmi trois plages d'intensités distinctes. Une plage d'intensités est destinée à prévenir l'apparition de cellules biologiques anormales telles que des cellules cancéreuses ; une plage est destinée à la détection de cellules biologiques anormales et une plage est destinée à l'élimination de telles cellules anormales et au traitement de la pathologie.

Comme indiqué plus haut, la plage d'intensités destinée à la prévention est de préférence comprise entre 0.05 et 0.2 W/cm². La plage d'intensités destinée à la détection est de préférence comprise entre 1 et 3 W/cm² et la plage d'intensités destinée au traitement est de préférence supérieure à 10 W/cm².

Si une base de connaissance suffisamment fiable est constituée, il est possible que les modes d'utilisation soient gérés de façon totalement autonome et automatique. Cela signifierait que la phase de détection est utilisée pour détecter la présence ou l'absence de cellules anormales, comme expliqué précédemment. Si aucune cellule anormale n'est détectée, la phase de prévention est activée, également comme décrit ci-dessus. Par contre, en cas de détection de cellules anormales, la phase de traitement est déclenchée de façon autonome, sans nécessairement l'intervention de personnel médical.

Selon un mode de réalisation avantageux, les différents modes d'utilisation sont gérés au moyen d'une application fonctionnant sur un dispositif externe et/ou au moyen d'un logiciel adapté. En principe, des droits différents sont attribués à différents intervenants. Par exemple, l'utilisateur est autorisé à introduire des informations personnelles et à se connecter de façon sécurisée à un compte. Il n'est par contre en principe pas autorisé à modifier des éléments liés aux différents cycles relatifs aux modes d'utilisation. Il peut être autorisé à consulter certaines informations.

Le personnel médical lui, peut être autorisé à agir sur les paramètres des cycles régissant les différents modes d'utilisation. Il peut également être autorisé à consulter des données telles que des images mémorisées ou des informations indiquant la présence ou l'absence de cellules anormales. La consultation ou la modification de paramètres, de valeurs ou d'autres éléments pourrait se faire soit localement, par exemple au moyen d'une connexion filaire ou d'une connexion sans fil à courte distance telle que par Bluetooth ou NFC ou au contraire, à distance. Cette connexion à distance pourrait se faire au moyen de dispositifs externes tels que des smartphones ou des dispositifs dédiés. Il est clair que la sécurité et la confidentialité des informations transitant par ces dispositifs doit être prise en compte.

La pièce d'habillement 10 de l'invention a été décrite notamment sous la forme d'un soutien-gorge. Elle pourrait être réalisée sous de nombreuses autres formes, comme par exemple sous toutes formes de sous-vêtements et notamment d'une chemisette, d'un débardeur, d'un collant, d'un slip, etc. La pièce d'habillement pourrait également être réalisée sous la forme d'un vêtement visible ou même éventuellement d'un survêtement tel qu'une guêtre, un pantalon, une veste, une manche, etc. Cette pièce d'habillement pourrait être utilisée à titre préventif et/ou curatif contre différentes formes de cancer telles que le cancer du sein, des testicules, du colon, etc., sans limitation à un type de cancer particulier. Les caractéristiques mentionnées ci-dessus en relation avec une pièce d'habillement sous forme de soutien-gorge s'appliquent également dans le cas où la pièce d'habillement est un slip ou un collant notamment.

La pièce d'habillement peut également être réalisée sous forme non pas de sous-vêtement, mais de vêtement visible. Une telle réalisation pourrait par exemple concerner un bonnet ou une cagoule, et être utilisée dans la prévention, la détection et/ou le traitement de carcinomes.

Le récepteur de commandes 15 permet également à une personne qui peut être l'utilisateur/utilisatrice ou un membre du personnel soignant, d'envoyer des commandes spécifiques au traitement souhaité.

Le récepteur de commandes 15 peut comporter un dispositif de réception sans fil ou un connecteur tel que par exemple un connecteur USB.

Le dispositif de mesure 17 peut être utilisé pour recevoir des informations concernant certains paramètres de l'utilisateur, tels que par exemple une température d'une certaine zone du corps de l'utilisateur. Ceci peut être réalisé par exemple au moyen d'une sonde de température.

Le résultat de ces mesures de paramètres peut être utilisé par exemple par un membre du personnel soignant pour adapter, modifier ou valider des modalités de traitement. Ces résultats de mesures peuvent également être pris en compte automatiquement pour adapter ces modalités de traitement.

Il est clair que cette pièce d'habillement pourrait être combinée à d'autres mécanismes de traitement conventionnels, en particulier dans le domaine du traitement du cancer.

## Revendications

1. Pièce d'habillement (10) destinée à être portée par un utilisateur de façon à couvrir une partie du corps de cet utilisateur, cette pièce d'habillement comportant un élément d'habillement souple (11) recouvrant ladite partie du corps de l'utilisateur, ladite pièce d'habillement comportant en outre un générateur (14) de signaux électriques et au moins un transducteur (12) solidaire dudit élément d'habillement souple (11), ledit au moins un transducteur (12) étant agencé pour convertir lesdits signaux électriques du générateur (14) de signaux électriques en ultrasons et pour transmettre les ultrasons générés par ce transducteur (12) à ladite partie du corps de l'utilisateur couverte par cet élément d'habillement souple (11), cette pièce d'habillement étant **caractérisée en ce que** ledit au moins un transducteur (12) est agencé pour émettre des ultrasons ayant au moins trois intensités acoustiques différentes comprises dans au moins trois plages d'intensités distinctes, l'une desdites plages d'intensité correspondant à un mode de prévention destiné à prévenir l'apparition de cellules biologiques anormales, une autre plage d'intensité correspondant à un mode de détection destiné à détecter de telles cellules anormales, et une troisième plage d'intensités correspondant à un mode de traitement destiné à l'élimination de telles cellules anormales et au traitement de la pathologie, **en ce que** cette pièce d'habillement comporte au moins un récepteur (12b) agencé pour recevoir des ondes provenant dudit au moins un transducteur (12) après réflexion par ladite partie du corps de l'utilisateur recouverte par ladite pièce d'habillement et pour transformer ces ondes en signaux électriques, **en ce que** cette pièce d'habillement comporte un module de communication (18) agencé pour transmettre ces signaux électriques à une unité de traitement (21) agencée pour en extraire une information, **en ce que** cette pièce d'habillement comporte un récepteur de commande (15) agencé pour recevoir un message de commande dépendant de ladite information extraite par l'unité de traitement (21) et pour transmettre ce message de commande audit générateur de signaux (14), ce générateur de signaux (14) étant agencé pour activer ledit au moins un transducteur (12) selon le contenu du message reçu, le mode de prévention étant activé si aucune cellule anormale n'est détectée lorsque ledit au moins un transducteur (12) est utilisé en mode de détection, le mode de traitement étant activé si des cellules anormales sont détectées lorsque ledit au moins un transducteur (12) est utilisé en mode de détection, et **en ce que** ces modes d'utilisation sont gérés de façon totalement autonome et automatique.

2. Pièce d'habillement selon la revendication 1, **caractérisée en ce qu'**elle comporte au moins trois transducteurs (12) d'au moins trois types différents, l'un des types de transducteurs étant agencé pour émettre des ultrasons ayant une intensité comprise dans une première plage d'intensités, un autre type de transducteurs étant agencé pour émettre des ultrasons ayant une intensité comprise dans une deuxième plage d'intensités distincte de ladite première plage d'intensités et un autre type de transducteurs étant agencé pour émettre des ultrasons ayant une intensité comprise dans une troisième plage d'intensités distincte desdites première et deuxième plage d'intensités.

3. Pièce d'habillement selon l'une quelconque des revendications précédentes, **caractérisée en ce que** les ultrasons d'une plage d'intensité ont une intensité acoustique comprise entre 0.05 et 0.2 W/cm².

4. Pièce d'habillement selon l'une quelconque des revendications précédentes, **caractérisée en ce que** les ultrasons d'une plage d'intensité ont une intensité acoustique comprise entre 1 et 3 W/cm².

5. Pièce d'habillement selon l'une quelconque des revendications précédentes, **caractérisée en ce que** les ultrasons d'une plage d'intensité ont une intensité acoustique supérieure à 10 W/cm².

6. Pièce d'habillement selon la revendication 1, **caractérisée en ce qu'**elle comporte un élément d'adaptation d'impédance (20) disposé entre ledit au moins un transducteur (12) et ladite partie du corps de l'utilisateur couverte par ledit l'élément d'habillement souple (11).

7. Pièce d'habillement selon la revendication 6, **caractérisée en ce que** l'élément d'adaptation d'impédance (20) est un élément en silicone.

8. Pièce d'habillement selon la revendication 6, **caractérisée en ce que** l'élément d'adaptation d'impédance (20) a une impédance acoustique comprise entre 1 et 20 MRayl.

9. Pièce d'habillement selon la revendication 1, **caractérisée en ce que** ledit au moins un récepteur (12b) est formé par ledit au moins un transducteur (12).

10. Pièce d'habillement selon la revendication 1, **caractérisée en ce que** la pièce d'habillement comporte un module de communication (18) destiné à transmettre à un dispositif externe, des données provenant dudit au moins un récepteur (12b).

## Patentansprüche

1. Kleidungsstück (10), das dazu bestimmt ist, von einem Benutzer getragen zu werden, um einen Körperteil dieses Benutzers zu bedecken, wobei das besagte Kleidungsstück ein flexibles Kleidungselement (11) umfasst, das den besagten Körperteil des Benutzers bedeckt, wobei das besagte Kleidungsstück ferner einen Generator (14) für elektrische Signale und mindestens einen Wandler (12) umfasst, der an dem besagten flexiblen Kleidungsstück (11) befestigt ist, wobei der besagte mindestens eine Wandler (12) so angeordnet ist, dass er die besagten elektrischen Signale von dem elektrischen Signalgenerator (14) in Ultraschall umwandelt und den von diesem Wandler (12) erzeugten Ultraschall an den besagten Körperteil des Benutzers überträgt, der von diesem flexiblen Kleidungselement (11) bedeckt ist, wobei dieses Kleidungsstück **dadurch gekennzeichnet ist, dass** der besagte mindestens eine Wandler (12) so angeordnet ist, dass er Ultraschall mit mindestens drei verschiedenen Schallintensitäten in mindestens drei verschiedenen Intensitätsbereichen aussendet, wobei einer der besagten Intensitätsbereiche einem Präventionsmodus entspricht, der dazu bestimmt ist, das Auftreten abnormaler biologischer Zellen zu verhindern, ein anderer Intensitätsbereich einem Erkennungsmodus entspricht, der dazu bestimmt ist, solche abnormalen Zellen zu erkennen, und ein dritter Intensitätsbereich einem Behandlungsmodus entspricht, der dazu bestimmt ist, solche abnormalen Zellen zu eliminieren und die Pathologie zu behandeln, dass dieses Kleidungsstück mindestens einen Empfänger (12b) umfasst, der so angeordnet ist, dass er Wellen von dem besagten mindestens einen Wandler (12) nach der Reflexion durch den von dem besagten Kleidungsstück bedeckten besagten Körperteil des Benutzers empfängt und diese Wellen in elektrische Signale umwandelt, dass dieses Kleidungsstück ein Kommunikationsmodul (18) umfasst, das so angeordnet ist, dass es diese elektrischen Signale an eine Verarbeitungseinheit (21) überträgt, die so angeordnet ist, dass sie daraus Informationen extrahiert, dass dieses Kleidungsstück einen Steuerungsempfänger (15) umfasst, der so angeordnet ist, dass er eine Steuerungsnachricht empfängt, die von den von der Verarbeitungseinheit (21) extrahierten besagten Informationen abhängt, und diese Steuerungsnachricht an den besagten Signalgenerator (14) überträgt, wobei dieser Signalgenerator (14) so angeordnet ist, dass er den besagten mindestens einen Wandler (12) entsprechend dem Inhalt der empfangenen Nachricht aktiviert, wobei der Präventionsmodus aktiviert wird, wenn keine abnormalen Zellen erkannt werden, wenn der besagte mindestens eine Wandler (12) im Erkennungsmodus verwendet wird, wobei der Verarbeitungsmodus aktiviert wird, wenn abnormale Zellen erkannt werden, wenn der besagte mindestens eine Wandler (12) im Erkennungsmodus verwendet wird, und dass diese Verwendungsmodi vollständig autonom und automatisch verwaltet werden.

2. Kleidungsstück nach Anspruch 1, **dadurch gekennzeichnet, dass** es mindestens drei Wandler (12) von mindestens drei verschiedenen Typen umfasst, wobei einer der Wandlertypen so angeordnet ist, dass er Ultraschall mit einer Intensität aussendet, die in einem ersten Intensitätsbereich liegt, ein anderer Wandlertyp so angeordnet ist, dass er Ultraschall mit einer Intensität aussendet, die in einem zweiten Intensitätsbereich liegt, der sich von dem besagten ersten Intensitätsbereich unterscheidet, und ein anderer Wandlertyp so angeordnet ist, dass er Ultraschall mit einer Intensität aussendet, die in einem dritten Intensitätsbereich liegt, der sich von dem besagten ersten und zweiten Intensitätsbereich unterscheidet.

3. Kleidungsstück nach einem der vorhergehenden Ansprüche, **dadurch gekennzeichnet, dass** der Ultraschall eines Intensitätsbereichs eine Schallintensität zwischen 0,05 und 0,2 W/cm² aufweist.

4. Kleidungsstück nach einem der vorhergehenden Ansprüche, **dadurch gekennzeichnet, dass** der Ultraschall eines Intensitätsbereichs eine Schallintensität zwischen 1 und 3 W/cm² aufweist.

5. Kleidungsstück nach einem der vorhergehenden Ansprüche, **dadurch gekennzeichnet, dass** der Ultraschall eines Intensitätsbereichs eine Schallintensität von mehr als 10 W/cm² aufweist.

6. Kleidungsstück nach Anspruch 1, **dadurch gekennzeichnet, dass** es ein Impedanzanpassungselement (20) umfasst, das zwischen dem besagten mindestens einen Wandler (12) und dem besagten Körperteil des Benutzers angeordnet ist, der von dem besagten flexiblen Kleidungselement (11) bedeckt ist.

7. Kleidungsstück nach Anspruch 6, **dadurch gekennzeichnet, dass** das Impedanzanpassungselement (20) ein Silikonelement ist.

8. Kleidungsstück nach Anspruch 6, **dadurch gekennzeichnet, dass** das Impedanzanpassungselement (20) eine akustische Impedanz zwischen 1 und 20 MRayl aufweist.

9. Kleidungsstück nach Anspruch 1, **dadurch gekennzeichnet, dass** der besagte mindestens ein Empfänger (12b) durch den besagten mindestens einen Wandler (12) gebildet ist.

10. Kleidungsstück nach Anspruch 1, **dadurch gekennzeichnet, dass** das Kleidungsstück ein Kommunikationsmodul (18) umfasst, das dazu bestimmt ist, von dem besagten mindestens einen Empfänger (12b) kommende Daten an eine externe Vorrichtung zu übertragen.

## Claims

1. An item of clothing (10) intended to be worn by a user so as to cover a part of the body of this user, this item of clothing comprising a flexible clothing element (11) covering said part of the body of the user, said item of clothing further comprising an electrical signal generator (14) and at least one transducer (12) integral with the said flexible clothing element (11), said at least one transducer (12) being arranged to convert said electrical signals from the electrical signal generator (14) into ultrasounds and to transmit the ultrasounds generated by said transducer (12) to said part of the user's body covered by said flexible clothing element (11), said item of clothing being **characterized in that** said at least one transducer (12) is arranged to emit ultrasounds having at least three different acoustic intensities comprised in at least three distinct intensity ranges, one of said intensity range corresponding to a prevention mode intended to prevent the apparition of abnormal biological cells, another intensity range corresponding to a detection mode intended to detect such abnormal cells, and a third intensity range corresponding to a treatment mode intended to eliminate such abnormal cells and to treat the pathology, **in that** the item of clothing comprises at least a receiver (12b) arranged to receive waves coming from said at least one transducer (12) after reflection by said part of the user's body covered by said item of clothing and to convert these waves into electrical signals, **in that** the item of clothing comprises a communication module (18) arranged for transmitting these electrical signals to a processing unit (21) arranged to extract an information, **in that** the item of clothing comprises an instruction receiver (15) arranged to receive an instruction message depending on said information extracted by the processing unit (21) and to transmit this instruction message to said signal generator (14), said signal generator (14) being arranged to activate said at least one transducer (12) according to the content of the message received, the prevention mode being activated if no abnormal cell is detected when said at least one transducer (12) is used in the detection mode, the treatment mode being activated if abnormal cells are detected when said at least one transducer (12) is used in the detection mode, and **in that** the modes of use are managed fully autonomously and automatically.

2. Item of clothing according to claim 1, **characterized in that** it comprises at least three transducers (12) of at least three different types, one of the types of transducers being arranged to emit ultrasounds having an intensity comprised in a first range of intensities, another type of transducers being arranged to emit ultrasounds having an intensity comprised in a second range of intensities distinct from said first range of intensities and another type of transducers being arranged to emit ultrasounds having an intensity comprised in a third range of intensities distinct from said first and second range of intensities.

3. Item of clothing according to any one of the preceding claims, **characterized in that** the ultrasounds of one intensity range have an acoustic intensity between 0.05 and 0.2 W/cm².

4. Item of clothing according to any of the preceding claims, **characterized in that** the ultrasounds of an intensity range have an acoustic intensity between 1 and 3 W/cm².

5. Item of clothing according to any one of the preceding claims, **characterized in that** the ultrasounds of an intensity range have an acoustic intensity greater than 10 W/cm².

6. Item of clothing according to claim 1, **characterized in that** it comprises an impedance matching element (20) disposed between said at least one transducer (12) and said part of the user's body covered by said flexible clothing element (11).

7. Item of clothing according to claim 6, **characterized in that** the impedance matching element (20) is a silicone element.

8. Item of clothing of claim 6, **characterized in that** the impedance matching element (20) has an acoustic impedance between 1 and 20 MRayl.

9. Item of clothing according to claim 1, **characterized in that** said at least one receiver (12b) is formed by said at least one transducer (12).

10. Item of clothing according to claim 1, **characterized in that** the item of clothing comprises a communication module (18) arranged for transmitting data from said at least one receiver (12b) to an external device.
